# EUROPEAN PATENT SPECIFICATION

(11) **EP 1 733 518 B1**
(45) Date of publication and mention of the grant of the patent: **11.12.2013**
(21) Application number: 05708995.5
(22) Date of filing: 11.03.2005
(51) Int. Cl.: H04W 64/00, G01S 5/00, H04W 4/00, H04W 88/06

(54) **POSITIONING OF WIRELESS MEDICAL DEVICES WITH SHORT-RANGE RADIO FREQUENCY TECHNOLOGY**
POSITIONIERUNG DRAHTLOSER MEDIZINISCHER EINRICHTUNGEN MIT KURZREICHWEITEN-HOCHFREQUENZTECHNOLOGIE
POSITIONNEMENT DE DISPOSITIFS MEDICAUX SANS FIL AVEC UNE TECHNOLOGIE DE RADIOFREQUENCES A COURTE PORTEE

(30) Priority: 31.03.2004 US 558279 P; 15.10.2004 US 619117 P
(43) Date of publication of application: 20.12.2006
(73) Proprietor: Koninklijke Philips N.V., 5656 AE Eindhoven (NL)
(72) Inventor: BALDUS, Heribert, NL-5621 BA Eindhoven (NL); KLABUNDE, Karin, NL-5621 BA Eindhoven (NL); MUESCH, Guido, NL-5621 BA Eindhoven (NL); SANTOS FARRAS, Joan, NL-5621 BA Eindhoven (NL)
(74) Representative: Verweij, Petronella Danielle
(86) International application number: PCT/IB2005/050881
(87) International publication number: WO 2005/096568

(56) References cited:
- EP-A- 1 239 634
- EP-A- 1 293 800
- WO-A-2004/054304
- US-A1- 2002 167 919

## Description

The following relates to the medical arts. It finds particular application in tracking equipment and personnel in hospitals and other medical facilities, and will be described with particular reference thereto. More generally, it finds application in tracking medical equipment, wireless medical sensor arrays, medical personnel wearing or carrying cellular telephones, personal data assistants, short-range wireless-equipped identification tags, and other wireless communication devices, and other mobile devices having short-range wireless connections in medical facility settings.

Hospitals and other medical facilities are rapidly moving toward wireless networking of medical diagnostic devices, medical treatment devices, and the like. Wireless networking enables rapid transmission of data to a nurses' station, floor monitor, or other central monitoring location, reduces the number of wires and cables in patients' rooms, and allows rapid hookup of devices to the medical facility network.

Additionally, it has been recognized that wireless networking provides convenient device tracking. For example, in the context of an IEEE 802.11 wireless hospital network infrastructure, the location of roaming network devices is already largely determined based on which wireless access point has the strongest communication link(s) with the roaming device. Existing network-infrastructure based tracking systems utilize and refine this built-in network tracking capability to track wireless devices that are compatible with the deployed wireless network infrastructure. Network tracking enables medical personnel to rapidly locate mobile wireless networked medical equipment, which saves time and manpower. In emergency medical situations the ability to rapidly locate networked medical equipment can save lives.

An exemplary network tracking system is described in EP 1 239 634 entitled "Hybrid Bluetooth/RFID based real time location tracking. The network tracking system described in this document comprises a plurality of distributed fixed devices communicating in two ways with mobile network devices. The first way of communication is based on a wireless communication protocol, such as Bluetooth. The second way of communication is based on RFID tags attached to the mobile network devices for reading by the readers mounted on fixed network devices.

These existing network tracking systems are limited to tracking wireless devices of their specific wireless technology. Local wireless devices employing Bluetooth, ZigBee, or other wireless technologies that are not network-compatible are not tracked. Such local wireless technologies are commonly used for short-range communication. For example, Bluetooth enabled cellular telephones, personal data assistants (PDA's), and the like are carried by many medical personnel. The Bluetooth connectivity enables these devices to communicate with the owner's personal computer or with other devices located in the same room or nearby. Bluetooth connections are also used to wirelessly connect medical probes, wireless vital signs sensor arrays, or the like with associated monitor devices. Bluetooth, ZigBee, and other local wireless links are generally limited to about ten meters or less, and generally cannot connect with the 802.11 wireless hospital network.

The following contemplates improved apparatuses and methods that overcome the aforementioned limitations and others.

According to one aspect, a tracking method is provided for tracking a local wireless device in a medical facility having a medical facility network which employs a wireless network communication protocol via a plurality of network access points distributed through the medical facility. The method comprises estimating locations of a plurality of wireless network devices that communicate with at least one of the plurality of network access points via the wireless network communication protocol, wherein the estimation of the locations is based on wireless network connections between the wireless network devices and the medical facility network. The local wireless device, which employs a non-network wireless communications protocol which is incompatible with the wireless network communication protocol of the medical facility network and is thus not able to communicate with any of the plurality of network access points, is detected.. A location of the local wireless device within the medical facility is estimated based on a local wireless communication via the non-network wireless communications protocol between the local wireless device and at least one nearby wireless network device, and on the location of the at least one nearby wireless network device, wherein the at least one nearby wireless network device employs the wireless network communication protocol and the non-network wireless communications protocol.

According to another aspect, a tracking system is disclosed for tracking a local wireless device in a medical facility having a medical facility network which employs a wireless network communication protocol via a plurality of network access points distributed through the medical facility wherein a plurality of wireless network devices are adapted to communicate with at least one of the plurality of network access points via the wireless network communication protocol. The tracking system comprises a network devices tracking system designed and arranged for tracking locations of at least the wireless network devicebased on wireless network connections between the wireless network devices and the medical facility network. The tracking system also comprises a local wireless device comprising local wireless communication hardware designed and arranged for employing a selected non-network local wireless communication protocol which is incompatible with the wireless network communication protocol of the medical facility network, such that the local wireless device is not able to communicate with any of the plurality of network access points. A means is provided for detecting the local wireless device, comprising local non-network wireless communication hardware installed in or integrated with at least one nearby wireless network device and employing the selected non-network local wireless communication protocol, such that the at least one nearby wireless network device employs the wireless network communication protocol and the non-network wireless communications protocol. A means is provided for estimating a location of the local wireless device within the medical facility based on the local non-network wireless communication between the local wireless device and the at least one nearby wireless network device, and on the location of the at least one nearby wireless network device.

One advantage resides in improved tracking for non-networked equipment, devices, personnel, and other items in a medical facility.

Another advantage resides in providing tracking of non-networked items in a medical facility without requiring additional communication hardware.

Yet another advantage resides in providing integrated tracking of both networked and non-networked items in a medical facility.

Numerous additional advantages and benefits will become apparent to those of ordinary skill in the art upon reading the following detailed description.

The invention may take form in various components and arrangements of components, and in various process operations and arrangements of process operations. The drawings are only for the purpose of illustrating preferred embodiments and are not to be construed as limiting the invention.
FIGURE 1 diagrammatically shows a tracking system for a hospital facility capable of tracking both networked and non-networked devices.
FIGURE 2 diagrammatically shows connections between the hospital facility network and network devices, and local wireless communication between wireless network devices and local wireless devices not connected with the hospital facility network.
FIGURE 3 diagrammatically illustrates estimating the location of a local wireless device not connected with the network based on signal strength of the local wireless communication.
FIGURE 4 shows a block diagram of a client program for detecting local wireless devices that is installed on each network device capable of local non-network wireless communication.

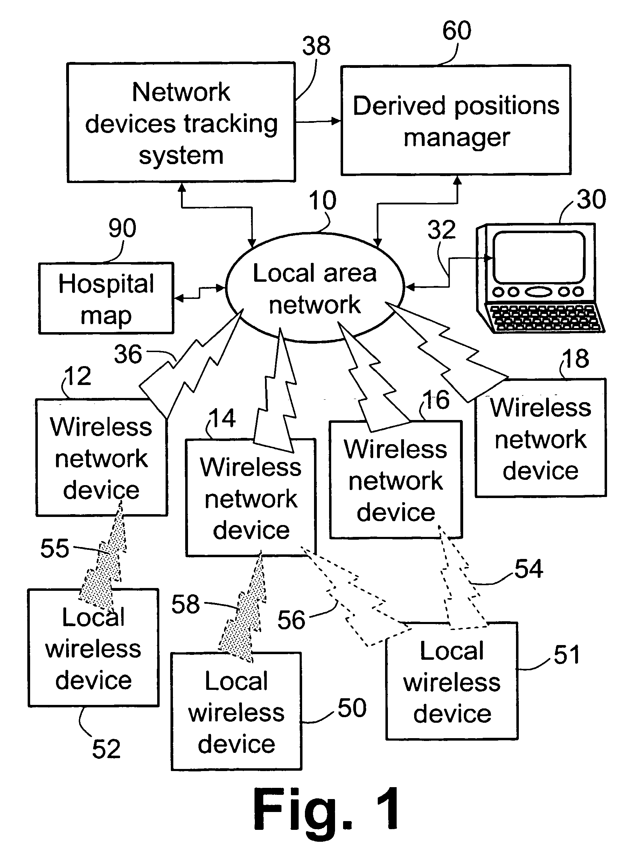

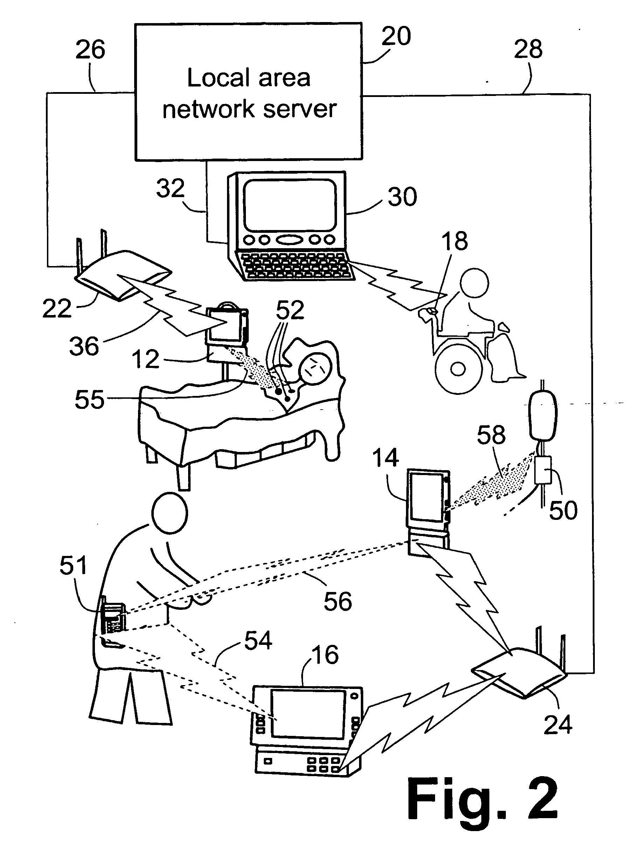

An illustrated tracking system is described with reference to FIGURE 1, which shows a diagrammatic block diagram of the system, and with reference to FIGURE 2, which illustrates example components of the medical facility network, example wireless network devices, and example local wireless devices. The hospital network includes a local area network **10** that wirelessly communicates with wireless network devices **12, 14, 16, 18**. For example, as shown in FIGURE 2, the wireless network devices may include a vital signs monitoring station **12**, a intravenous (IV) injection flow controller **14**, a patients' monitor at a nurses' station **16**, a vital signs transducer **18** carried by an ambulating patient, and so forth. The illustrated wireless network devices **12, 14, 16, 18** are examples, and other devices can be similarly wirelessly connected with the local area network **10**.

The local area network **10** typically includes a local area network server **20** and a plurality of wireless network access points **22, 24** distributed through the medical facility and connected with the local area network server **20** by wired Ethernet cables **26, 28**. Alternatively, a Token Ring or another wired network connection can be employed. The network access points **22, 24** are distributed throughout the medical facility to provide substantially complete wireless network coverage for the facility, so that wireless network devices can communicate with the network **10** from substantially anywhere in the medical facility. Although not shown, in some networks wireless access extension points are provided which wirelessly communicate with one of the wired network access points **22, 24** to provide extended network area coverage.

Network devices such as the vital signs monitoring station **12**, the IV flow controller **14**, and the nurses' station monitor **16** wirelessly communicate with the nearest or most strongly wirelessly coupled network access points **22, 24** in order to wirelessly connect with the medical facility network **10**. Additionally or alternatively, one or more software access points can be employed. For example, a computer **30** connected with the local area network server **20** by wired Ethernet cable **32** includes a software access point implemented by a wireless card (not shown) installed in the computer **30** and suitable software. Network devices such as the vital signs transducer **18** may wirelessly connect with the software access point embodied by the computer **30** in order to wirelessly connect with the medical facility network **10**.

The wireless network connections are indicated in FIGURES 1 and 2 by jagged connectors drawn using solid lines, such as the example labeled connector **36** connecting the vital signs monitor station **12** with the access point **22** of the medical facility network **10**. (The remaining wireless network connections are similarly indicated by solid-line jagged connectors, but are not labeled with reference numbers in FIGURES 1 and 2). In some embodiments, the wireless network communication comports with an IEEE 802.11 local area network protocol; however, other wireless local area network protocols can be employed.

Some of the wireless network devices are roaming network devices. For example, the ambulatory vital signs transducer **18** moves frequently as the ambulatory patient roams the halls of the medical facility, and the IV flow controller **14** is frequently moved from room to room. Other wireless devices are less frequently roaming or may even be substantially stationary. For example, the vital signs monitor station **12** may generally stay in the same patient's room for several days, and the nurses' station monitor **16** in some medical facilities may not move for years. Network devices which are not wireless, such as the computer **30**, generally move infrequently or not at all.

A network devices tracking system **38** tracks the mobile or potentially mobile wireless network devices **12, 14, 16, 18**. The network devices tracking system **38** is typically a software program or module executing on the local area network server **20**; however, another computer or other digital processing hardware can embody the network devices tracking system **38**.

As a roaming wireless network device moves, its distance from the locked-in access point used for network communication changes. If the roaming wireless network device moves far enough, it may be switched over to another access point which is now closer. For example, if the ambulatory patient carrying the vital signs transducer **18** moves away from the computer **30** (which embodies a software access point) and toward the access point **24**, eventually its wireless network connection will switch over to the access point **24.** Thus, the wireless network inherently includes the capability of performing a rough tracking of roaming network devices. The wireless network devices tracking system **38** more precisely determines the location of each wireless network device **12, 14, 16, 18** based on factors such as signal strength, triangulation of the signals received by several access points, or so forth. The wireless network devices tracking system 38 is limited to tracking network devices **12, 14, 16, 18** which are connected with the medical facility network **10**.

With continuing reference to FIGURES 1 and 2, not all wireless devices are network devices connected with the medical facility network **10**. For example, local wireless devices **50, 51, 52** may include local wireless capability that is not compatible with the 802.11 wireless protocol or other wireless network protocol employed by the medical facility network **10**. As illustrated in FIGURE 2, the local wireless device **50** is an infusion pump controlling an intravenous flow. The infusion pump **50** is wirelessly connected with the IV flow controller **14** which controls the infusion pump **50** to set the rate of intravenous infusion. The local wireless device **51** is a cellular telephone carried by a doctor or other medical person.

In some cases, the local wireless device can be an autonomous sensor network. For example, an autonomous vital signs sensor network **52** includes wireless sensors monitoring a patient's electrocardiograph (ECG), pulse rate, blood oxygen, and so forth. The vital signs monitor station **12** receives and displays the vital signs measured by the vital signs sensor network **52**; however, the vital signs sensor network **52** is autonomous and continues to monitor the vital signs of the patient even if the patient is taken outside of radio range of the monitor station **12**, for example to be taken to a diagnostic imaging facility. Other examples of wireless devices that may be present in medical facility settings include wireless patient and staff identification bracelets or tags that include short-range wireless transmitters that identify the patient or staff member to local monitors.

The local wireless devices **50, 51, 52** employ a non-network wireless communication protocol such as an IEEE 802.15.1 protocol (also sometimes called Bluetooth), an IEEE 802.15.3 protocol, an 802.15.4 protocol (also sometimes called ZigBee), or the like. Bluetooth, ZigBee, and most other short-range wireless communication protocols are incompatible with the 802.11 network protocol and other wireless network protocols. (The cellular telephone **51** is a local wireless device insofar as it includes short-range wireless protocol capability such as Bluetooth or Zigbee. The cellular telephone **51** also inherently includes longer-range wireless transmission capability in order to communicate with the cellular telephone network; however, the cellular protocol generally is incompatible with the 802.11 network protocol and most other wireless network protocols, and so the cellular telephone **51** is not connected with the wireless medical facility network **10**.) However, the local wireless devices **50, 51, 52** can communicate with each other when the distance between devices is within the short range of the local wireless communication capability, which is typically less than ten meters.

To locate these non-network local wireless devices **50, 51, 52,** the network devices **12, 14, 16, 18**, and optionally also other non-network local wireless devices, are used. In addition to being wirelessly connected with the medical facility network **10**, many of these network devices include Bluetooth, ZigBee, or other non-network wireless connectivity, and various such network devices are generally present throughout the medical facility. Accordingly, each network device **12, 14, 16, 18** having the requisite non-network local wireless communication capability performs occasional scans to detect local wireless devices within range that are also capable of communicating by the non-network local wireless communication protocol. In the illustrated configuration, the vital signs monitor station **12** detects the vital signs sensor array **52.** The IV flow controller **14** detects both the infusion pump **50** and the cellular telephone **51**. The nurses' station monitor **16** detects only the cellular telephone **51**. The vital signs transducer **18** does not detect any of the local wireless devices **50, 51, 52**. Because Bluetooth, ZigBee, and other non-network local wireless communication protocols are short-range, only nearby network devices are able to detect a given local wireless device. Moreover, some network devices may not have the capability to communicate using the requisite non-network local wireless communication protocol, and thus cannot detect even nearby local wireless devices.

The non-network local wireless communications are indicated in FIGURES 1 and 2 by jagged connectors **54, 55, 56, 58** drawn using dashed lines. Wireless communication **54** is between the nurses' station monitor **16** and the cellular telephone **51**. Wireless communication **55** is between the vital signs monitor station **12** and the sensors network **52**. Wireless communication **56** is between the IV flow controller **14** and the cellular telephone **51.** Wireless communication **58** is between the infusion pump **50** and the IV flow controller **14**. These Bluetooth, ZigBee, or other local wireless communications **54**, **55, 56, 58** are to be distinguished from wireless network connections following the 802.11 protocol or another wireless network protocol, such as the wireless network connection **36**, which are indicated by jagged connectors drawn using solid lines. Moreover, it is to be appreciated that the illustrated local wireless communications **54, 55, 56, 58** are a snapshot in time - as devices move about, communications may be dropped as communicating devices move apart from one another, or added as devices capable of local wireless communication come together.

As illustrated in FIGURES 1 and 2, some local wireless communications **54, 56** are brief polling communications limited to detecting and identifying local wireless devices within range. These are typically not considered to be wireless connections, but rather are brief wireless polling communications. On the other hand, in some cases a network device may actually be wirelessly connected with a local wireless device for the purpose of substantive transmission of data, control commands, or the like. In FIGURES 1 and 2, two example wireless connections are: (i) the local wireless communication **58** between the infusion pump **50** and the IV flow controller **14**; and (ii) the local wireless communication **55** between the autonomous vital signs sensors network **52** and the vital signs monitor station **12**. The substantive and prolonged nature of these local wireless connections **55, 58** are indicated by filling the corresponding jagged connectors with a dotted pattern in FIGURES 1 and 2.

When nearby wireless network devices **12, 14, 16** detect local wireless devices **50, 51, 52**, they communicate information about the detected local wireless devices to a derived positions manager **60**. Typically, the communicated information includes an identification of the local wireless devices **50, 51, 52**, which may for example be a media access control (MAC) address for each detected local wireless device. Optionally, an indication of the signal strength of the local wireless communication **54, 55, 56, 58** is also communicated. Based on the local wireless communication **54, 55, 56, 58** and information indicative of the location of the detecting nearby wireless network devices **14, 16**, the location of the detected local wireless devices **50, 51, 52** can be estimated. Optionally, local wireless communication between two or more of the local wireless devices **50, 51, 52** (not illustrated) can also be used to aid in the locating.

Location information for roaming wireless network devices is suitably obtained from the network devices tracking system **38**. In a medical facility employing a mostly wireless network infrastructure, the tracked wireless network devices may be sufficient to perform locating of the local wireless devices. On the other hand, if there are a significant number of wired network devices that are generally stationary, the location of these stationary network devices is optionally also retrieved from a data file into which location information for stationary network devices has been previously entered. In this case, those stationary network devices which include Bluetooth, ZigBee, or another local wireless communication capability can also be used for detecting and localizing local wireless devices. The derived positions manager **60** estimates the location of detected local wireless devices **50, 51, 52** based on the locations of the nearby detecting network devices **12, 14, 16** using any suitable locating method. Other *a priori* knowledge, such as room layouts, last known position and device portability, nature of the device (i.e., sensors **52** that remain associated with the same patient), and the like can also be used by the derived positions manager **60** in locating the local wireless devices.

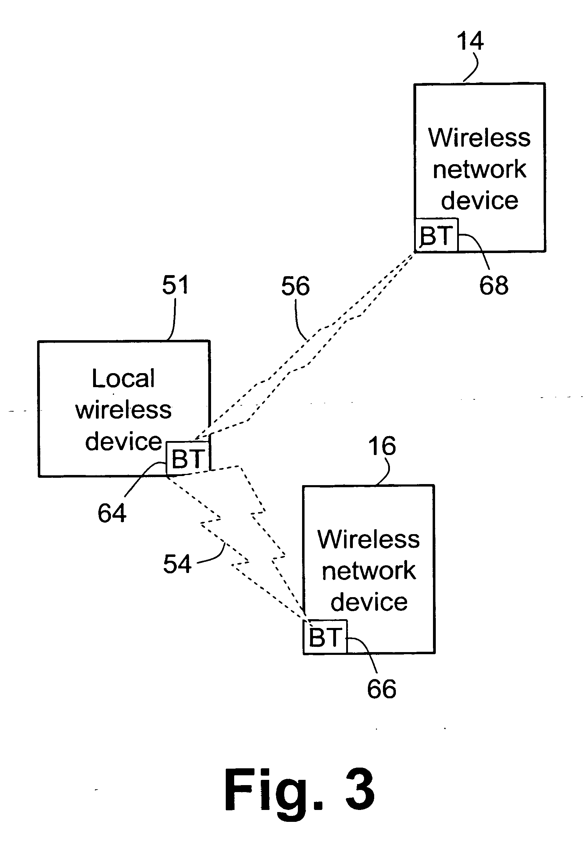

With reference to FIGURE 3, one suitable method for estimating the location of a local wireless device based on local wireless communication is described. Specifically, the location of the cellular telephone **51** is estimated based on the local wireless communications **54, 56**. The local wireless communication **54** between the cellular telephone **51** and the nurses' station monitor **16** is achieved using: (i) local wireless communication hardware **64** installed in or integrated with the cellular telephone **51** employing a selected non-network local wireless communication protocol such as Bluetooth or ZigBee; and (ii) local non-network wireless communication hardware **66** installed in or integrated with the nurses' station monitor **16** employing the same selected non-network local wireless communication protocol. Similarly, the local wireless communication **56** between the cellular telephone **51** and the IV flow controller **14** is achieved using: (i) the local wireless communication hardware **64** of the cellular telephone **51**; and (ii) local non-network wireless communication hardware **68** installed in or integrated with the IV flow controller **14** employing the same selected non-network local wireless communication protocol. In FIGURE 3, all three local non-network wireless communication hardware components **64, 66, 68** are Bluetooth cards installed in the respective devices **51, 16, 14**.

In some embodiments, the derived positions manager **60** estimates the location of the local wireless cellular telephone **51** based on the signal strengths of the local wireless communications **54, 56**. In the example illustrated in FIGURE 3, the signal strength of the local wireless communications **54** is substantially stronger than the signal strength of the local wireless communications **56**, as indicated diagrammatically by a thicker jagged connector representation of the wireless communication **54**. This stronger signal for the wireless communication **54** indicates that the distance between the cellular telephone **51** and the nurses' station monitor **16** is smaller than the distance between the cellular telephone **51** and the IV flow controller **14**. Accordingly, the cellular telephone **51** can be estimated to be relatively closer to the nurses' station monitor **16** and relatively further away from the IV flow controller **14**. In some embodiments, a more quantitative distance estimate is made, for example based on the assumption that the signal strength for an omni-directional wireless communication decreases proportional to the square of the distance from the signal source.

If the signal strength is not measured or is not communicated to the derived positions manager **60**, the distance between the local wireless device and a nearby detecting network device can be estimated as less than or about the maximum communication distance of the local wireless communication. This approach is simple and can be relatively accurate when the range of the local wireless communication is short. For example, Bluetooth and ZigBee devices typically have a communication range of about ten meters, enabling localization to within about ten meters by this method. If only a single network device detects the local wireless device, then it may be sufficient to estimate the location of the local wireless device as substantially coinciding with the location of the detecting network device, which will be accurate to within about ten meters for typical Bluetooth and ZigBee communication ranges.

If a local wireless device is detected by more than one network device (such as the cellular telephone **51** being detected by two network devices **14, 16**) then the combination can be analyzed to more closely estimate the location of the local wireless device. For example, a triangulation method can be employed, in which a circle or sphere around each network device is estimated having a radius corresponding to the determined distance between the network device and the local wireless device, and the intersection of the circles or spheres is the estimated location of the local wireless device.

In yet another approach by which the derived positions manager **60** can estimate the location of a detected local wireless device, if there is a substantive local wireless connection between the local wireless device and a network device, such as the illustrated local wireless connections **55, 58**, then it is typically reasonable to assume that the local wireless device is in the same room as the connected network device. For example, it is highly likely that the vital signs sensor network **52** is in the same room as the vital signs monitor station **12** when the sensor network **52** is wirelessly connected with the monitor station **12** to perform vital signs monitoring of a patient.

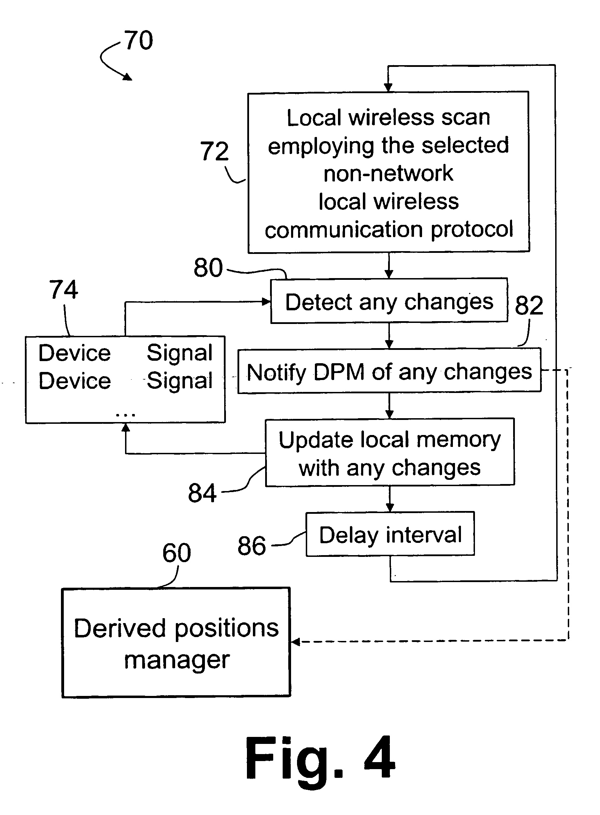

With reference to FIGURE 4, in one suitable embodiment a client program **70** is installed on each of the network devices **12, 14, 16, 18** that includes the capability to communicate using the Bluetooth, ZigBee, or other local wireless communication protocol. The client program **70** performs operations that scan for local wireless devices and communicate detection of such devices to the derived positions manager **60**. In an operation **72**, the client program **70** causes the network device to employ the Bluetooth, ZigBee, or other selected non-network local wireless communication protocol to scan for other devices having the requisite Bluetooth, ZigBee, or other local wireless communication capability. The detection results are compared with previous detection results stored in a local memory **74** on the network device in a comparison process operation **80**. In the illustrated example, the local memory **74** stores a device identification, such as a MAC address, and the signal strength of the local wireless communication for each detected local wireless device. The comparison process operation **80** detects addition of a new local wireless device (possibly indicating that a roaming local wireless device has moved into range), disappearance of a previously detected local wireless device (possibly indicating that the roaming local wireless device has moved out of range), or a change in the signal strength of the local wireless communication (possibly indicating movement of the local wireless device). If a change is detected, the derived positions manager **60** is notified of the change in operation **82**, and the local memory **74** is updated in operation **84**. After a delay **86**, the process operations **72, 80, 82, 84** are repeated. In this way, any change in the detection status of any local wireless device within the range of the network device is timely reported via wireless connection to the derived positions manager **60**; however, when nothing has changed, no wireless network communication traffic is generated.

The use of the client program **70** installed on each of the network devices **12, 14, 16, 18** advantageously reduces wireless network traffic and can speed up the local wireless polling operation **72**. However, in some other contemplated embodiments the derived positions manager performs centralized polling by individually commanding each network device to perform polling and report results to the derived positions manager. The derived positions manager performs the change detection based on the data received from the network device. These embodiments do not require installing remote client software on the network devices.

With returning reference to FIGURE 1, the location information can be provided to the user in any suitable manner. In some embodiments, a digital hospital map **90** is accessed via the local area network **10** and displayed on the computer 30 or on another display device. The location of each detected local wireless device estimated by the derived positions manager **60** is suitably superimposed on the displayed hospital map. Instead of or in addition to such a graphical representation, the information can be listed; for example, each local wireless device can be listed along with the room in which that device is disposed. Moreover, optionally the locations of wireless network devices determined by the network devices tracking system **38** is integrated with the locations of the local wireless devices to generate an integrated database of device locations that can be mapped or otherwise displayed.

Relating a location to the digital hospital map **90** can also be used to determine which room of the medical facility contains the location. This type of analysis can be used by the derived positions manager **60** to determine which room contains the nearby network devices detecting a particular local wireless device. If all the detecting nearby network devices are in the same room, the local wireless device is suitably estimated to also be located in that room. Similarly, where a local wireless device has an established local wireless connection with a network device, the room containing the connected network device is also generally a good estimate of the location of the wirelessly connected local wireless device.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims.

## Claims

1. A tracking method for tracking a local wireless device in a medical facility having a medical facility network (10) which employs a wireless network communication protocol via a plurality of network access points (22, 24) distributed through the medical facility, the tracking method comprising:
estimating locations of a plurality of wireless network devices (12, 14, 16, 18) that communicate with at least one of the plurality of network access points (22, 24) via the wireless network communication protocol wherein the estimation of the locations is based on wireless network connections (36) between the wireless network devices and the medical facility network (10);
detecting a local wireless device (50, 51, 52) which employs a non-network wireless communications protocol which is incompatible with the wireless network communication protocol of the medical facility network (10) and is thus not able to communicate with any of the plurality of network access points (22, 24); and
estimating a location of the local wireless device (50, 51, 52) within the medical facility (10) based on a local wireless communication via the non-network wireless communications protocol between the local wireless device (50, 51, 52) and at least one nearby wireless network device (12, 14, 16, 18), and on the location of the at least one nearby wireless network device, wherein the at least one nearby wireless network device employs the wireless network communication protocol and the non-network wireless communications protocol.

2. The tracking method as set forth in claim 1, wherein the wireless network connection (36) comports with an IEEE 802.11 based wireless protocol.

3. The tracking method as set forth in claim 1, wherein the local wireless communication (54, 55, 56, 58) between the local wireless device (50, 51, 52) and the at least one nearby wireless network device (12, 14, 16) employs at least one of: (i) an IEEE 802.15.1 wireless protocol; and (ii) an 802.15.4 wireless protocol.

4. The tracking method as set forth in claim 1, wherein, prior to the detecting, the local wireless device (50, 52) and the nearby wireless network device (12, 14) establish a local wireless communication connection (55, 58) therebetween, and the estimating includes:
estimating the location of the local wireless device (50, 52) as substantially coinciding with the location of the nearby wireless network device (12, 14).

5. The tracking method as set forth in claim 1, wherein the estimating includes:
estimating a distance between the local wireless device (50, 51, 52) and the at least one nearby wireless network device (12, 14, 16) based on a strength of a wireless signal employed in the local wireless communication (54, 55, 56, 58).

6. The tracking method as set forth in claim 1, wherein the at least one nearby wireless network device includes a plurality of nearby network devices (14, 16) that detect the local wireless device (51), each of which employs the wireless network communication protocol and the non-network wireless communications protocol, and the estimating includes:
estimating a distance between the local wireless device (51) and each nearby network device (14, 16) based on the local wireless communication (54, 56); and
estimating a location of the local wireless device (51) based on the estimated distances and the locations of the nearby network devices (14, 16).

7. The tracking method as set forth in claim 1, wherein the estimating includes:
estimating a distance between the local wireless device (50, 51, 52) and the at least one nearby wireless network device (12, 14, 16) based on a maximum communication distance of the local wireless communication (54, 55, 56, 58).

8. The tracking method as set forth in claim 1, wherein the estimating includes:
estimating the location of the local wireless device (50, 52) as being within a room of the medical facility containing the nearby wireless network device (12, 14).

9. The tracking method as set forth in claim 1, further including:
repeating the detecting;
during a subsequent detecting, identifying a change in the local wireless communication (54, 55, 56, 58) between the local wireless device (50, 51, 52) and the at least one nearby wireless network device (12, 14, 16); and
updating the location of the local wireless device (50, 51, 52) within the medical facility based on the change.

10. The tracking method as set forth in claim 9, wherein the change includes one of:
loss of local wireless communication between the local wireless device (50, 51, 52) and at least one nearby wireless network device (12, 14, 16) during the subsequent detecting, and
pickup of a new local wireless communication between the local wireless device (50, 51, 52) and a network device (18) other than the at least one nearby wireless network device (12, 14, 16) of the initial detecting, wherein the other network device employs the wireless network communication protocol and the non-network wireless communications protocol.

11. The tracking method as set forth in claim 1, wherein (i) the at least one nearby wireless network device (12, 14, 16) is part of a plurality of wireless network devices (12, 14, 16, 18) wirelessly connected with the medical facility network (10), (ii) the information indicative of the location of the at least one nearby wireless network device is part of information indicative of the locations of the plurality of wireless network devices, and (iii) the tracking method further includes:
repeating the detecting and estimating for a plurality of local wireless devices (50, 51, 52) not connected with the medical facility network (10) to estimate a location for each local wireless device; and
combining the estimated locations of the plurality of local wireless devices (50, 51, 52) and the information indicative of the locations of the plurality of wireless network devices (12, 14, 16, 18) to generate an integrated database of device locations.

12. The tracking method as set forth in claim 1, further including:
superimposing the estimated location of the local wireless device (50, 51, 52) on a map (90) of at least a portion of the medical facility to provide a visual indication of the estimated location.

13. A tracking system for tracking a local wireless device in a medical facility having a medical facility network (10) which employs a wireless network communication protocol via a plurality of network access points (22, 24) distributed through the medical facility wherein a plurality of wireless network devices (12, 14, 16, 18) are adapted to communicate with at least one of the plurality of network access points (22, 24) via the wireless network communication protocol, the tracking system comprising:
a network devices tracking system (38) designed and arranged for tracking locations of at least the wireless network devices (12, 14, 16, 18) based on wireless network connections (36) between the wireless network devices (12, 14, 16, 18) and the medical facility network (10);
a local wireless device (51) comprising local wireless communication hardware designed and arranged for employing a selected non-network local wireless communication protocol which is incompatible with the wireless network communication protocol of the medical facility network (10), such that the local wireless device (51) is not able to communicate with any of the plurality of network access points (22, 24);
a means (64, 66, 68, 70) for detecting the local wireless device (50, 51, 52), comprising local non-network wireless communication hardware (66, 68) installed in or integrated with at least one nearby wireless network device (14, 16) and employing the selected non-network local wireless communication protocol, such that the at least one nearby wireless network device employs the wireless network communication protocol and the non-network wireless communications protocol; and
a means (60) for estimating a location of the local wireless device (50, 51, 52) within the medical facility based on the local non-network wireless communication (54, 55, 56, 58) between the local wireless device (50, 51, 52) and the at least one nearby wireless network device (12, 14, 16, 18), and on the location of the at least one nearby wireless network device (12, 14, 16).

14. The tracking system as set forth in claim 13, wherein the selected non-network local communication protocol is one of: (i) an 802.15.1 protocol, and (ii) an 802.15.4 protocol.

15. The tracking system as set forth in claim 13, wherein the means (64, 66, 68, 70) for detecting the local wireless device (50, 51, 52) further includes:
client software (70) installed on the at least one nearby wireless network device (12, 14, 16) that causes the at least one nearby wireless network device to scan for other devices in range that are capable communicating using the selected non-network local communication protocol.

16. The tracking system as set forth in claim 13, further including:
a digital map (90) of the medical facility; and
a graphical display (30) capable of displaying the digital map with the estimated location of the local wireless device (50, 51, 52) superimposed thereon.

## Patentansprüche

1. Trackingverfahren zur Verfolgung eines lokalen drahtlosen Gerätes in einer medizinischen Einrichtung mit einem Netzwerk (10) der medizinischen Einrichtung, das ein Kommunikationsprotokoll für drahtlose Netze über eine Vielzahl von Netzwerkzugangspunkten (22, 24) nutzt, die über die medizinische Einrichtung verteilt sind, wobei das Trackingverfahren Folgendes umfasst:
Ermitteln von Standorten einer Vielzahl von drahtlosen Netzwerkgeräten (12, 14, 16, 18), die zumindest mit einem der Vielzahl von Netzwerkzugangspunkten (22, 24) über das Kommunikationsprotokoll des drahtlosen Netzes kommunizieren, wobei die Ermittlung der Standorte auf drahtlosen Netzwerkverbindungen (36) zwischen den drahtlosen Netzwerkgeräten und dem Netzwerk (10) der medizinischen Einrichtung basiert,
Erkennen eines lokalen drahtlosen Gerätes (50, 51, 52), das ein drahtloses, nicht netzwerkgebundenes Kommunikationsprotokoll nutzt, das nicht mit dem Kommunikationsprotokoll des drahtlosen Netzes (10) der medizinischen Einrichtung kompatibel und somit nicht in der Lage ist, mit einem der Vielzahl von Netzwerkzugangspunkten (22, 24) zu kommunizieren, und
Ermitteln eines Standortes des lokalen drahtlosen Gerätes (50, 51, 52) innerhalb der medizinischen Einrichtung (10) zwischen dem lokalen drahtlosen Gerät (50, 51, 52) und zumindest einem in der Nähe befindlichen drahtlosen Netzwerkgerät (12, 14, 16, 18) anhand lokaler drahtloser Kommunikation über das nicht netzwerkgebundene drahtlose Kommunikationsprotokoll und des Standorts des zumindest einen in der Nähe befindlichen drahtlosen Netzwerkgeräts, wobei das zumindest eine in der Nähe befindliche drahtlose Netzwerkgerät das Kommunikationsprotokoll für drahtlose Netze und das drahtlose, nicht netzwerkgebundene Kommunikationsprotokoll nutzt.

2. Trackingverfahren nach Anspruch 1, wobei die drahtlose Netzwerkverbindung (36) mit einem drahtlosen Protokoll gemäß IEEE 802.11 kompatibel ist.

3. Trackingverfahren nach Anspruch 1, wobei die lokale drahtlose Kommunikation (54, 55, 56, 58) zwischen dem lokalen drahtlosen Gerät (50, 51, 52) und dem zumindest einen in der Nähe befindlichen drahtlosen Netzwerkgerät (12, 14, 16) zumindest entweder (i) ein Protokoll für drahtlose Netze gemäß IEEE 802.15.1 oder (ii) ein Protokoll für drahtlose Netze gemäß IEEE 802.15.4 nutzt.

4. Trackingverfahren nach Anspruch 1, wobei das lokale drahtlose Gerät (50, 52) und das in der Nähe befindliche drahtlose Netzwerkgerät (12, 14) vor der Erkennung eine lokale drahtlose Kommunikationsverbindung (55, 58) untereinander aufbauen und die Ermittlung Folgendes umfasst:
Ermitteln des Standorts des lokalen drahtlosen Gerätes (50, 52) als im Wesentlichen mit dem Standort des in der Nähe befindlichen drahtlosen Netzwerkgerätes (12, 14) übereinstimmend.

5. Trackingverfahren nach Anspruch 1, wobei die Ermittlung Folgendes umfasst:
Ermitteln einer Entfernung zwischen dem lokalen drahtlosen Gerät (50, 51, 52) und dem zumindest einen in der Nähe befindlichen drahtlosen Netzwerkgerät (12, 14, 16) anhand der Stärke eines drahtlosen Signals, das in der lokalen drahtlosen Kommunikation (54, 55, 56, 58) genutzt wird.

6. Trackingverfahren nach Anspruch 1, wobei das zumindest eine in der Nähe befindliche drahtlose Netzwerkgerät eine Vielzahl von in der Nähe befindlichen Netzwerkgeräten (14, 16) umfasst, die das lokale drahtlose Gerät (51) erkennen, wobei jedes von ihnen das Kommunikationsprotokoll für drahtlose Netze und das drahtlose, nicht netzwerkgebundene Kommunikationsprotokoll nutzt und die Ermittlung Folgendes umfasst:
Ermitteln der Entfernung zwischen dem lokalen drahtlosen Gerät (51) und jedem in der Nähe befindlichen Netzwerkgerät (14, 16) anhand der lokalen drahtlosen Kommunikation (54, 56) und
Ermitteln des Standorts des lokalen drahtlosen Gerätes (51) anhand der ermittelten Entfernungen und der Standorte der in der Nähe befindlichen Netzwerkgeräte (14, 16).

7. Trackingverfahren nach Anspruch 1, wobei die Ermittlung Folgendes umfasst:
Ermitteln der Entfernung zwischen dem lokalen drahtlosen Gerät (50, 51, 52) und dem zumindest einen in der Nähe befindlichen drahtlosen Netzwerkgerät (12, 14, 16) anhand eines maximalen Kommunikationsabstands der lokalen drahtlosen Kommunikation (54, 55, 56, 58).

8. Trackingverfahren nach Anspruch 1, wobei die Ermittlung Folgendes umfasst:
Ermitteln des Standorts des lokalen drahtlosen Geräts (50, 52) als in einem Raum der medizinischen Einrichtung befindlich, der das in der Nähe befindliche drahtlose Netzwerkgerät (12, 14) enthält.

9. Trackingverfahren nach Anspruch 1, das ferner Folgendes umfasst:
Wiederholen der Erkennung,
während einer nachfolgenden Erkennung Identifizieren einer Veränderung innerhalb der lokalen drahtlosen Kommunikation (54, 55, 56, 58) zwischen dem lokalen drahtlosen Gerät (50, 51, 52) und dem zumindest einen in der Nähe befindlichen drahtlosen Netzwerkgerät (12, 14, 16) und
Aktualisieren des Standorts des lokalen drahtlosen Geräts (50, 51, 52) innerhalb der medizinischen Einrichtung anhand der Veränderung.

10. Trackingverfahren nach Anspruch 9, wobei die Veränderung eine der folgenden Aspekte umfasst:
Ausfall der lokalen drahtlosen Kommunikation zwischen dem lokalen drahtlosen Gerät (50, 51, 52) und zumindest einem in der Nähe befindlichen drahtlosen Netzwerkgerät (12, 14, 16) während der nachfolgenden Erkennung und
Aufnahme einer neuen lokalen drahtlosen Kommunikation zwischen dem lokalen drahtlosen Gerät (50, 51, 52) und einem anderen Netzwerkgerät (18) als dem zumindest einen in der Nähe befindlichen drahtlosen Netzwerkgerät (12, 14, 16) der anfänglichen Erkennung, wobei das andere Netzwerkgerät das Kommunikationsprotokoll für drahtlose Netze und das drahtlose, nicht netzwerkgebundene Kommunikationsprotokoll nutzt.

11. Trackingverfahren nach Anspruch 1, wobei (i) das zumindest eine in der Nähe befindliche drahtlose Netzwerkgerät (12, 14, 16) Teil einer Vielzahl von drahtlosen Netzwerkgeräten (12, 14, 16, 18) ist, die drahtlos mit dem Netzwerk (10) der medizinischen Einrichtung verbunden sind, (ii) die Informationen, die den Standort des zumindest einen in der Nähe befindlichen drahtlosen Netzwerkgerätes angeben, Teil von Informationen sind, die die Standorte der Vielzahl von drahtlosen Netzwerkgeräten angeben, und (iii) das Trackingverfahren ferner Folgendes umfasst:
Wiederholen der Erkennung und Ermittlung für eine Vielzahl von lokalen drahtlosen Geräten (50, 51, 52), die nicht mit dem Netzwerk (10) der medizinischen Einrichtung verbunden sind, um den Standort für jedes lokale drahtlose Gerät zu ermitteln, und
Kombinieren der ermittelten Standorte der Vielzahl von lokalen drahtlosen Geräten (50, 51, 52) und der Informationen, die die Standorte der Vielzahl von drahtlosen Netzwerkgeräte (12, 14, 16, 18) angeben, um eine integrierte Datenbank mit Gerätestandorten zu erstellen.

12. Trackingverfahren nach Anspruch 1, das ferner Folgendes umfasst:
Überlagern des ermittelten Standorts des lokalen drahtlosen Geräts (50, 51, 52) auf einen Plan (90) zumindest eines Teils der medizinischen Einrichtung zur Bereitstellung einer optischen Anzeige des ermittelten Standorts.

13. Trackingsystem zur Verfolgung eines lokalen drahtlosen Gerätes in einer medizinischen Einrichtung mit einem Netzwerk (10) der medizinischen Einrichtung, das ein Kommunikationsprotokoll für drahtlose Netze über eine Vielzahl von Netzwerkzugangspunkten (22, 24) nutzt, die über die medizinische Einrichtung verteilt sind, wobei eine Vielzahl von drahtlosen Netzwerkgeräten (12, 14, 16, 18) für die Kommunikation mit zumindest einem der Vielzahl von Netzwerkzugangspunkten (22, 24) über das Kommunikationsprotokoll für drahtlose Netze ausgelegt ist, wobei das Trackingsystem Folgendes umfasst:
ein Trackingsystem (38) für Netzwerkgeräte, das für die Verfolgung von Standorten zumindest der drahtlosen Netzwerkgeräte (12, 14, 16, 18) anhand von drahtlosen Netzwerkverbindungen (36) zwischen den drahtlosen Netzwerkgeräten (12, 14, 16, 18) und dem Netzwerk (10) der medizinischen Einrichtung konzipiert und ausgelegt ist,
ein lokales drahtloses Gerät (51), das lokale Hardware für drahtlose Kommunikation umfasst, die für die Nutzung eines ausgewählten lokalen, drahtlosen, nicht netzwerkgebundenen Kommunikationsprotokolls ausgelegt ist, das mit dem Kommunikationsprotokoll für das drahtlose Netzwerk (10) der medizinischen Einrichtung nicht kompatibel ist, sodass das lokale drahtlose Gerät (51) nicht in der Lage ist, mit einem der Vielzahl von Netzzugangspunkten (22, 24) zu kommunizieren,
Mittel (64, 66, 68, 70) zur Erkennung des lokalen drahtlosen Geräts (50, 51, 52), die lokale nicht netzwerkgebundene Hardware (66,68) für drahtlose Kommunikation umfassen, die in zumindest ein in der Nähe befindliches drahtloses Netzwerkgerät (14,16) eingebaut oder integriert ist und das ausgewählte lokale, drahtlose, nicht netzwerkgebundene Kommunikationsprotokoll nutzt, sodass das zumindest eine in der Nähe befindliche drahtlose Netzwerkgerät das Kommunikationsprotokoll für drahtlose Netze und das nicht netzwerkgebundene, drahtlose Kommunikationsprotokoll nutzt, und
Mittel (60) zur Ermittlung eines Standorts des lokalen drahtlosen Geräts (50, 51, 52) innerhalb der medizinischen Einrichtung anhand der lokalen, nicht netzwerkgebundenen drahtlosen Kommunikation (54, 55, 56, 58) zwischen dem lokalen drahtlosen Gerät (50, 51, 52) und dem zumindest einen in der Nähe befindlichen drahtlosen Netzwerkgerät (12, 14, 16, 18) und des Standorts des zumindest einen in der Nähe befindlichen drahtlosen Netzwerkgerätes (12, 14, 16).

14. Trackingsystem nach Anspruch 13, wobei das ausgewählte, nicht netzwerkgebundene lokale Kommunikationsprotokoll entweder (i) ein Protokoll gemäß 802.15.1 oder (ii) ein Protokoll gemäß 802.15.4 ist.

15. Trackingsystem nach Anspruch 13, wobei die Mittel (64, 66, 68, 70) zur Erkennung des lokalen drahtlosen Geräts (50, 51, 52) ferner Folgendes umfassen:
Client-Software (70), die in dem zumindest einen in der Nähe befindlichen drahtlosen Netzwerkgerät (12, 14, 16) installiert ist und das zumindest eine in der Nähe befindliche drahtlose Netzwerkgerät veranlasst, nach anderen Geräten im Bereich zu suchen, die in der Lage sind, unter Verwendung des ausgewählten nicht netzwerkgebundenen lokalen Kommunikationsprotokolls zu kommunizieren.

16. Trackingsystem nach Anspruch 13, das ferner Folgendes umfasst:
einen digitalen Plan (90) der medizinischen Einrichtung und
eine grafische Anzeige (30), die in der Lage ist, den digitalen Plan mit dem überlagerten ermittelten Standort des lokalen drahtlosen Gerätes (50, 51, 52) anzuzeigen.

## Revendications

1. Procédé de suivi pour suivre un dispositif sans fil local dans une installation médicale ayant un réseau d'installation médicale (10) qui utilise un protocole de communication à réseau sans fil via une pluralité de points d'accès de réseau (22, 24) distribués dans l'installation médicale, le procédé de suivi comprenant :
l'estimation d'emplacements d'une pluralité de dispositifs de réseau sans fil (12, 14, 16, 18) qui communiquent avec au moins l'un de la pluralité des points d'accès de réseau (22, 24) via le protocole de communication de réseau sans fil dans lequel l'estimation d'emplacements se base sur les connexions de réseau sans fil (36) entre les dispositifs de réseau sans fil et le réseau de l'installation médicale (10) ;
la détection d'un dispositif sans fil local (50, 51, 52) qui utilise un protocole de communication sans fil non réseau qui est incompatible avec le protocole de communication à réseau sans fil de l'installation médicale (10) et ne peut donc pas communiquer avec l'un quelconque de la pluralité de points d'accès de réseau (22, 24) ; et
l'estimation d'un emplacement du dispositif sans fil local (50, 51, 52) dans l'installation médicale (10) sur la base d'une communication sans fil locale via le protocole de communication sans fil non réseau entre le dispositif sans fil local (50, 51, 52) et au moins un dispositif à réseau sans fil proche (12, 14, 16, 18) et sur l'emplacement de l'au moins un dispositif à réseau sans fil proche, dans lequel l'au moins un dispositif à réseau sans fil proche utilise le protocole de communication à réseau sans fil et le protocole de communication sans fil non réseau.

2. Procédé de suivi tel que défini dans la revendication 1, dans lequel la connexion à réseau sans fil (36) comporte un protocole sans fil basé sur IEEE 802.11.

3. Procédé de suivi tel que défini dans la revendication 1, dans lequel la communication sans fil locale (54, 55, 56, 58) entre le dispositif sans fil local (50, 51, 52) et l'au moins un dispositif à réseau sans fil proche (12, 14, 16) utilise au moins l'un parmi : (i) un protocole sans fil IEEE 802.15.1 ; et (ii) un protocole sans fil 802.15.4.

4. Procédé de suivi tel que défini dans la revendication 1, dans lequel, avant la détection, le dispositif sans fil local (50, 52) et le dispositif à réseau sans fil proche (12, 14) établissent une connexion de communication sans fil locale (55, 58) entre eux et l'estimation comprend :
l'estimation de l'emplacement du dispositif sans fil local (50, 52) comme coïncidant substantiellement avec l'emplacement du dispositif de réseau sans fil proche (12, 14).

5. Procédé de suivi tel que défini dans la revendication 1, l'estimation comprenant :
l'estimation d'une distance entre le dispositif sans fil local (50, 51, 52) et l'au moins un dispositif de réseau sans fil proche (12, 14, 16) sur la base d'une puissance d'un signal sans fil utilisé dans la communication sans fil locale (54, 55, 56, 58).

6. Procédé de suivi tel que défini dans la revendication 1, dans lequel l'au moins un dispositif de réseau sans fil proche comprend une pluralité de dispositifs de réseau proches (14, 16) qui détectent le dispositif sans fil local (51), chacun d'eux utilisant le protocole de communication à réseau sans fil et le protocole de communication sans fil non réseau et l'estimation comprend :
l'estimation d'une distance entre le dispositif sans fil local (51) et chaque dispositif de réseau proche (14, 16) sur la base de la communication sans fil locale (54, 56) ; et
l'estimation d'un emplacement du dispositif sans fil local (51) sur la base des distances estimées et des emplacements des dispositifs de réseau proches (14, 16).

7. Procédé de suivi tel que défini dans la revendication 1, dans lequel l'estimation comprend :
l'estimation d'une distance entre le dispositif sans fil local (50, 51, 52) et l'au moins un dispositif de réseau sans fil proche (12, 14, 16) sur la base d'une distance de communication maximale de la communication sans fil locale (54, 55, 56, 58).

8. Procédé de suivi tel que défini dans la revendication 1, dans lequel l'estimation comprend :
l'estimation de l'emplacement du dispositif sans fil local (50, 52) comme étant dans une pièce de l'installation médicale contenant le dispositif de réseau sans fil proche (12, 14).

9. Procédé de suivi tel que défini dans la revendication 1, comprenant en outre :
la répétition de la détection ;
pendant une détection consécutive, l'identification d'un changement dans la communication sans fil locale (54, 55, 56, 58) entre le dispositif sans fil local (50, 51, 52) et l'au moins un dispositif de réseau sans fil proche (12, 14, 16) ; et
la réactualisation de l'emplacement du dispositif sans fil local (50, 51, 52) dans l'installation médicale sur la base du changement.

10. Procédé de suivi tel que défini dans la revendication 9, dans lequel le changement comprend l'un parmi :
la perte d'une communication sans fil locale entre le dispositif sans fil local (50, 51, 52) et au moins un dispositif de réseau sans fil proche (12, 14, 16) pendant la détection consécutive, et
la récupération d'une nouvelle communication sans fil locale entre le dispositif sans fil local (50, 51, 52) et un dispositif de réseau (18) autre que l'au moins un dispositif de réseau sans fil proche (12, 14, 16) de la détection initiale, dans lequel l'autre dispositif de réseau utilise le protocole de communication du réseau sans fil et le protocole de communication sans fil non réseau.

11. Procédé de suivi tel que défini dans la revendication 1, dans lequel (i) l'au moins un dispositif de réseau sans fil proche (12, 14, 16) fait partie d'une pluralité de dispositifs de réseau sans fil (12, 14, 16, 18) raccordé sans fil avec le réseau de l'installation médicale (10), (ii) l'information indicative de l'emplacement de l'au moins un dispositif de réseau sans fil proche fait partie de l'information indicative des emplacements de la pluralité de dispositifs de réseau sans fil et (iii) le procédé de suivi comprend en outre :
la répétition de la détection et de l'estimation d'une pluralité de dispositifs sans fil locaux (50, 51, 52) non raccordés au réseau de l'installation médicale (10) pour estimer un emplacement pour chaque dispositif sans fil local ; et
la combinaison des emplacements estimés de la pluralité de dispositifs sans fil locaux (50, 51, 52) et l'information indicative des emplacements de la pluralité de dispositifs de réseau sans fil (12, 14, 16, 18) pour générer une base de données intégrée d'emplacements de dispositif.

12. Procédé de suivi tel que défini dans la revendication 1, comprenant en outre :
la superposition de l'emplacement estimé du dispositif sans fil local (50, 51, 52) sur une carte (90) d'au moins une partie de l'installation médicale pour fournir une indication visuelle de l'emplacement estimé.

13. Système de suivi pour le suivi d'un dispositif sans fil local dans une installation médicale ayant un réseau d'installation médicale (10) qui utilise un protocole de communication de réseau sans fil via une pluralité de points d'accès de réseau (22, 24) distribués dans l'installation médicale, dans lequel une pluralité de dispositifs de réseau sans fil (12, 14, 16, 18) est adaptée pour communiquer avec au moins l'un de la pluralité des points d'accès de réseau (22, 24) via le protocole de communication de réseau sans fil, le système de suivi comprenant :
un système de suivi de dispositifs de réseau (38) conçu et disposé pour le suivi des emplacements d'au moins les dispositifs de réseau sans fil (12, 14, 16, 18) sur la base des connexions de réseau sans fil (36) entre les dispositifs de réseau sans fil (12, 14, 16, 18) et le réseau d'installation médicale (10) ;
un dispositif sans fil local (51) comprenant un matériel de communication sans fil local conçu et disposé pour utiliser un protocole de communication sans fil local non réseau choisi qui est incompatible avec le protocole de communication de réseau sans fil du réseau de l'installation médicale (10) de telle sorte que le dispositif sans fil local (51) ne puisse pas communiquer avec l'un quelconque de la pluralité de points d'accès de réseau (22, 24) ;
un moyen (64, 66, 68, 70) de détection du dispositif sans fil local (50, 51, 52), comprenant un matériel de communication sans fil non réseau local (66, 68) installé dans ou intégré avec au moins un dispositif de réseau sans fil proche (14, 16) et utilisant le protocole de communication sans fil local non réseau choisi de telle sorte que l'au moins un dispositif de réseau sans fil proche utilise le protocole de communication de réseau sans fil et le protocole de communication sans fil non réseau ; et
un moyen (60) pour estimer un emplacement du dispositif sans fil local (50, 51, 52) dans l'installation médicale sur la base de la communication sans fil non réseau locale (54, 55, 56, 58) entre le dispositif sans fil local (50, 51, 52) et l'au moins un dispositif de réseau sans fil proche (12, 14, 16, 18) et sur l'emplacement de l'au moins un dispositif de réseau sans fil proche (12, 14, 16).

14. Système de suivi tel que défini dans la revendication 13, dans lequel le protocole de communication local non réseau choisi est l'un parmi : (i) un protocole 802.15.1 et (ii) un protocole 802.15.4.

15. Système de suivi tel que défini dans la revendication 13, dans lequel le moyen (64, 66, 68, 70) pour détecter le dispositif sans fil local (50, 51, 52) comprend en outre :
un logiciel client (70) installé sur l'au moins un dispositif de réseau sans fil proche (12, 14, 16) qui entraîne le balayage par l'au moins un dispositif de réseau sans fil proche pour déterminer d'autres dispositifs de la série qui sont capables de communiquer en utilisant le protocole de communication local non réseau choisi.

16. Système de suivi tel que défini dans la revendication 13, comprenant en outre :
une carte numérique (90) de l'installation médicale ; et
un affichage graphique (30) capable d'afficher la carte numérique avec l'emplacement estimé du dispositif sans fil local (50, 51, 52) superposé sur celle-ci.
